## Europäisches Patentamt
## European Patent Office
## Office européen des brevets

(19)

(11) Numéro de publication: **0 117 860**
**B2**

(12) **NOUVEAU FASCICULE DE BREVET EUROPÉEN**

(45) Date de publication du nouveau fascicule du brevet:
**31.05.89**

(51) Int. Cl.⁴: **A 61 L 2/20**

(21) Numéro de dépôt: **84870011.8**

(22) Date de dépôt: **27.01.84**

(54) Procédé et appareil de stérilisation au moyen d'un agent gazeux.

(30) Priorité: **28.01.83 BE 209987**

(43) Date de publication de la demande:
**05.09.84 Bulletin 84/36**

(45) Mention de la délivrance du brevet:
**11.06.86 Bulletin 86/24**

(45) Mention de la decision concernant l'opposition:
**31.05.89 Bulletin 89/22**

(84) Etats contractants désignés:
**AT BE CH DE FR GB IT LI LU NL SE**

(56) Documents cité:
**DE-A-2 313 484**
**US-A-3 958 935**

**PHARMAZIE 31, 47 (1976), pp. 491-492**
**APPLIED MICROBIOLOGY, 25 (2) Feb. 1973, p. 205-207**
**APPLIED MICROBIOLOGY, Nov. 1966, 14 (6), p. 989-992**
**DRÄGERHEFT, no 174, pages 2654-2665 (1934)**
**Mode d'emploi de "Desinfektionskammer ASEPTOR", no 6751 (08/1971)**

(73) Titulaire: **UNIVERSITE CATHOLIQUE DE LOUVAIN, Place de l'Université, 1, B-1348 Louvain la Neuve (BE)**

(72) Inventeur: **Hennebert, Pierre, Rue des Ménapiens, 15, B-1040 Bruxelles (BE)**
Inventeur: **Gillard, Jean, Rue Leemans, 2, B-5991 Tourinnes- la- Grosse (BE)**
Inventeur: **Roland, Michel, Tomberg, 38, B-1200 Bruxelles (BE)**

(74) Mandataire: **Grisar, Daniel, c/o Office Kirkpatrick 4, square de Meeûs, B-1040 Bruxelles (BE)**

EP 0 117 860 B2

## Description

La présente invention concerne un procédé de sterilisation gazeuse pour le traitement de matières, d'objets ou d'enceintes avec un ou des produits chimiques à l'état gazeux.

Le recours à la stérilisation gazeuse est indispensable pour stériliser des produits ou des objets ne pouvant pas être soumis à des températures élevées telles qu'elles sont utilisées classiquement dans les méthodes de stérilisation thermique, à des rayonnements hautement énergétiques tels que les rayons gamma ou encore à des solutions germicides telles que les solutions de glutaraldéhyde.

D'une façon générale, la stérilisation gazeuse est principalement mise en oeuvre par l'exposition des surfaces à steriliser à des gaz biocides tels l'oxyde d'éthylrène ou le formaldéhyde. Les procédés actuellement utilisés présentent de nombreux inconvénients.

L'oxyde d'éthylène, qui est utilisé sur une très large échelle, réagit lentement. Hautement inflammable, il est par ailleurs très toxique, pouvant provoquer des effets mutagènes chez les personnes exposées. Il doit être utilisé à des concentrations élevées (de 500 à 2000 mg par litre) qui aggravent les risques (présence de résidus dans les objets, taux élevés dans l'atmosphère des ateliers de stérilisation, pollution de l'environnement).

La formaldéhyde, dont l'action biocide est connue depuis longtemps, présente nettement moins de risques que l'oxyde d'éthylène, mais les résultats de son utilisation suivant les procédés actuellement utilisés sont très peu satisfaisants, si, dans certaines conditions de travail, des cycles de stérilisation se sont révélés efficaces vis-à-vis de populations de spores connues pour leur résistance (B. subtilis, B. stearothermophilus) et directement exposées aux vapeurs de formaldéhyde, il faut reconnaître l'inconstance des résultats et les divergences de vues qui séparent les utilisateurs de ces méthodes. Aussi, des spécialistes en matière d'hygiène s'interrogent pour savoir si les appareils et procédés existant actuellement permettent d'obtenir une stérilisation ou, plus précisément, une désinfection. Le formaldéhyde est généralement utilisé de deux façons, à savoir soit en dépolymérisant le paraformaldéhyde par chauffage, soit en injectant du formol (solution aqueuse de formaldéhyde à environ 40 % additionnée de 5 à 10 % de méthanol) dans un stérilisateur alimenté, par ailleurs, en vapeur d'eau saturée sous pression subatmosphérique. Bien que le formaldéhyde ait une activité bocide très efficace à des concentrations supérieures à 5 mg par litre, voire 2 mg par litre, la stérilité n'est pas toujours atteinte dans ces conditions d'utilisation qui permettent difficilement de maîtriser les paramètres de stérilisation, notamment la concentration en gaz et l'humidité relative.

Ce caractère aléatoire de la stérilisation par le fomaldéhyde en présence de vapeur d'eau est connu depuis longtemps, comme il ressort d'une étude commencée en 1950 en Angleterre sous les auspices du Central Public Health Laboratory. En effet, il y est precisé dans la discussion des résultats expérimentaux qu'il a été difficile d'obtenir des résultats reproductibles, que l'effet de l'humidité a été équivoque et enfin que la désinfection par la vapeur de formaldéhyde n'est pas à essayer lorsqu'un autre procédé quelconque est disponible.

Par ailleurs, le rôle néfaste des condensations d'eau a été mis en évidence aussi.

La publication "Drägerheft, n° 174, pages 2654 - 2665, 1934" contient la description d'un appareillage pour la désinfection de masques à gaz et objets analogues, et d'un procédé selon lequel il fonctionne. L'atmosphère d'une armoire de désinfection est mise en circulation à travers ladite armoire puis à travers un conduit dans lequel elle est mise en contact avec un agent désinfectant (formaldéhyde) placé dans une ampoule. Du fait de la circulation réalisée, l'agent désinfectant est propulsé dans le circuit. Des moyens (ventilateur, spirale de chauffage) permettent de régler la quantité d'agent désinfectant libéré et mis en circulation.

Le mode d'emploi "Desinfektionskammer Aseptor" publié en août 1971, sous le n° 6751 par Drägerwerke A.G., décrit un dispositif analogue, dont le fonctionnement est basé sur la circulation d'une atmosphère gazeuse dans une enceinte, laquelle atmosphère vient lécher une source de formaldéhyde, sous forme de solution.

Cartains constructeurs d'apparails pour la stérilisation à basse température ont essayé d'éviter les problèmes de condensation d'eau an réalisant une purge automatique des condensats au cours du cycle de stérilisation. D'autres, conscients de la forte variation de la teneur en formaldéhyde gazeux lors du cycle de stérilisation, ont proposé des injections répétées de formaldéhyde en solution.

La présente invention a pour but de procurer un procédé et un appareil pour la stérilisation par un agent biocide gazeux, en particulier le formaldéhyde, qui évitent les inconvénients des techniques connues à ce jour.

La présente invention a pour objet un procédé de stérilisation d'une matière ou d'un objet par la mise en contact de cette matière ou de cet objet avec un mélange de gaz contenant au moins un agent stérilisant obtenu par évaporation d'une solution liquide, procédé suivant lequel la matière ou l'objet à stériliser et le mélange de gaz contenant au moins un agent stérilisant sont confinés dans une chambre étanche aux fluides, le mélange de gaz contenant au moins un agent stérilisant est propulsé en circuit et ramené en contact avec la solution liquide qui contient l'agent stérilisant à l'état dissous, liquide et/ou solide, en concentration suffisante pour établir la concentration désirée en agent stérilisant évaporé, et le mélange de gaz confiné dans la chambre est soumis à des variations de pression qui sont supérieures aux seules différences de pression nécessaires à la propulsion en circuit du mélange de gaz contenant au moins un agent stérilisant.

Avantageusement, l'agent stérilisant est le formaldéhyde.

De manière préférée, la solution liquide est une solution aqueuse de formaldéhyde, contenant éventuellement d'autres composants tels que des sels minéraux ou organiques, des polyols comme la glycérine, le propylèneglycol, l'éthylèneglycol ou des polyoxyéthylèneglycols.

Suivant une forme d'exécution avantageuse du procédé de l'invention, la température du mélange de gaz contenant au moins un agent stérilisant se situe entre 20 et 100°C, et de préférence entre 55 et 65°C, et la matière ou l'objet à stériliser sont portés, avant la mise an contact avec ce mélange de gaz à une température proche de celle de ce mélange de gaz ou égale à celle-ci.

Pour l'exécution du procédé, une part au moins des parties de la chambre en contact avec le mélange de gaz contenant au moins un agent stérilisant a de préférence été à la température de ce mélange de gaz.

En particulier, les opérations du procédé peuvent être exécutées avec des baisses de pression lusqu'à environ 1/20 de la pression atmosphérique et des augmentations de pression rétablissant la pression atmosphérique ou menant même jusqu'à 5 fois la pression atmosphérique. En général, il est cependant avantageux d'exécuter le procédé à des pressions qui ne dépassent pas sensiblement la pression atmosphérique.

Suivant un mode d'exécution avantageux de mélange de gaz confiné dans la chambre est soumis une ou plusieurs fois, au cours de l'exécution du procédé à une augmentation de pression, par introduction dans la chambre d'un gaz ou d'un mélange de gaz vecteur.

On entend ici par "gaz vecteur", un agent gazeux qui en soi, est inerte du point de vue de la stérilisation, mais dont l'adjonction au mélange de gaz présent dans la chambre permet d'y augmenter la pression totale.

En particulier, à cet effet, le gaz ou mélange de gaz vecteur est l'azote ou l'air ou un melange de ceux-ci. Suivant une forme d'exécution particulière du procédé conforme à l'invention,
- on introduit la matière ou l'objet à stériliser dans la chambre,
- on amène cette chambre à une première pression subatmosphérique,
- on introduit de la vapeur d'eau à une température égale ou voisine de celle à laquelle on veut porter la matière ou l'objet, jusqu'à obtention de la température voulue, une succion partielle hors de la chambre de cette vapeur et de l'eau condensée étant continuellement effectuée,
- on interrompt l'admission de vapeur d'eau,
- on amène la chambre à une seconde pression subatmosphérique jusqu'à disparition de l'eau qui s'est condensée lors de l'introduction de vapeur d'eau,
- on met la chambre en communication avec la solution maintenue à une température constante, égale à la température choisie pour le mélange de gaz qui est obtenu par évaporation de cette solution,
- on fait circuler le mélange de gaz contenu dans la chambre en le ramenant de manière continue en contact avec la solution,
- après que la teneur an agent stérilisant du mélange de gaz contenu dans la chambre s'est mise en équilibre avec la solution, on introduit dans la chambre un gaz ou un mélange de gaz vecteur, en y augmentant ainsi la pression jusqu'à obtention d'une pression totale inférieure, égale ou supérieure à la pression atmosphérique,
- on expose ansi la surface de la matière ou de l'objet pendant le temps requis pour obtenir la stérilité, tandis que le mélange de gaz contenu dans la chambre est maintenu en circulation et ramené en contact de manière continue avec la solution,
- on coupe la communication entre la solution et le mélange de gaz contenu dans la chambre,
- on élimine l'agent stérilisant contenu dans la chambre,
- on ramène l'intérieur de la chambre à la pression atmosphérique, et
- on en retire la matière ou l'objet.

Suivant une autre forme d'exécution du procédé comforme à l'invention,
- on introduit la matière ou l'objet à stériliser dans la chambre,
- on amène cette chambre à une première pression subatmosphérique,
- on introduit de la vapeur d'eau à une température égale ou voisine de celle à laquelle on veut porter la matière ou l'objet, jusqu'à obtention de la température voulue, une succion partielle hors de la chambre de cette vapeur et de l'eau condensée étant continuellement effectuée,
- l'introduction de la vapeur d'eau restant possible, on introduit une ou plusieurs fois dans la chambre un gaz ou mélange de gaz vecteur, jusqu'à obtention d'une pression totale inférieure, égale ou supérieure à la pression atmosphérique et après chaque introduction de gaz ou mélange de gaz vecteur, on ramène la chambre à une pression subatmosphérique inférieure à celle qu'on y à obtenue par l'introduction du gaz ou mélange de gaz vecteur,
- on interrompt l'admission de vapeur d'eau,
- on amène la chambre à une seconde pression subatmosphérique jusqu'à disparition de l'eau qui s'est condensée lors de l'introduction de vapeur d'eau,
- on met la chambre en communication avec la solution mantanue à une température constante, égale à la température choisie pour le mélange de gaz qui est obtenu par évaporation de cette solution,
- on fait circuler le mélange de gaz contenu dans la chambre en le ramenant de manière continue en contact avec la solution,

- après que la teneur en agent stérilisant du mélange de gaz contenu dans la chambre s'est mise en équilibre avec la solution, on introduit dans la chambre un gaz ou un mélange de gaz vecteur, en y augmentant ainsi la pression jusqu'à obtention d'une pression totale inférieure, égale ou supérieure à la pression atmosphérique,

- on expose ainsi la surface de la matière ou de l'objet pendant le temps requis pour obtenir la stérilité, tandis que le mélange de gaz contenu dans la chambre est maintenu en circulation et ramené en contact de manière continue avec la solution,

- on coupe la communication entre la solution et le mélange de gaz contenu dans la chambre,

- on élimine l'agent stérilisant contenu dans la chambre,

- on ramène l'intérieur de la chambre à la pression atmosphérique, et

- on en retire la matière ou l'objet.

L'invention a également pour objet des appareils pour l'exécution du procédé dans ses différentes formes de réalisation.

De manière particulière, l'invention a pour objet un appareil pour la stérilisation de matières ou d'objets par mise on contact avec un mélange de gaz contenant au moins un agent stérilisant obtenu par évaporation d'une solution liquide, cet appareil comprenant une chambre de traitement étanche aux fluides, à régulation thermostatique, munie d'au moins une porte conçue pour charger des matières ou objets dans la chambre et les en retirer, cet appareil comprenant on outre:

- un générateur d'agent stérilisant apte à contenir une solution de cet agent stérilisant et à évaporer cette solution à une température prédéterminée, grâce à des moyens de chauffage et/ou refroidissement et des moyens de régulation thermique,

- des moyens aptes à introduire le mélange de gaz produit par le générateur d'agent stérilisant dans la chambre, à faire circuler le mélange de gaz contenu dans la chambre et à le ramener en contact avec la solution contenue dans le générateur d'agent stérilisant,

- un générateur de vide raccordé à la chambre,

- un circuit d'admission de gaz vecteur raccordé à la chambre et comprenant un moyen de stérilisation d'un gaz et un moyen de chauffage thermostatique de ce gaz,

- un générateur de vapeur d'eau qui est raccordé d'une part à la chambre et d'autre part à une alimentation en eau et qui est pourvu d'un système de régulation de la température et/ou de la pression,

- des moyens aptes à mettre le générateur d'agent stérilisant, le générateur de vide, le circuit d'admission de gaz vecteur et le générateur de vapeur d'eau en communication avec la chambre ou à couper ces communications.

Suivant une forme de réalisation de cet appareil, la chambre est incluse dans une enveloppe propre à la circulation d'un fluide calorifique et raccordée à des moyens aptes à assurer la circulation, le chauffage et la régulation du chauffage du fluide calorifique.

Suivant une autre forme de réalisation de cet appareil, la chambre est une chambre en matière plastique non conductrice de l'électricité qui résiste aux efforts dûs aux pressions subatmosphériques et qui est munie d'organes de chauffage électriques intérieurs et/ou noyés dans la masse des parois.

Dans un appareil suivant l'invention, le moyen de chauffage thermostatique du gaz dans le circuit d'admission de gaz vecteur peut être un échangeur de chaleur chauffé par le générateur de vapeur d'eau.

Suivant une forme d'exécution avantageuse de l'appareil, la chambre est munie d'une sonde thermique raccordée à un dispositif de régulation qui fait persister le fonctionnement du générateur de vapeur d'eau au moins jusqu'à acquisition par la charge d'une température prédéterminée.

L'invention a également pour objet un appareil pour la stérilisation de matières ou d'objets par mise en contact avec un mélange de gaz contenant au moins un agent stérilisant obtenu par évaporation d'une solution liquide, cet appareil consistant en un tunnel à régulation thermostatique qui est pourvu d'un transporteur apte à y faire défiler les matières ou objets et qui comprend, depuis son entrée jusqu'à sa sortie:

- une section de préchauffage pourvue de moyens aptes à porter les matières ou objets à une température prédéterminée;

- une section de protection d'entrée pourvue de moyens aptes à y créer un flux d'air, préchauffé à une température prédéterminée, dirigée de manière à empêcher que le mélange gazeux contenu dans la section suivante du tunnel (section de stérilisation) ne puisse s'échapper par l'entrée du tunnel,

- une section de stérilisation qui est équipée d'un générateur d'agent stérilisant apte à contenir une solution de cet agent stérilisant et à évaporor cette solution à une température prédéterminée, grâce à des moyens de chauffage et/ou de refroidissement et des moyens de régulation thermique, et qui est également équipée de moyens aptes à introduire le mélange de gaz produit par le générateur d'agent stérilisant dans la section de stérilisation du tunnel, à faire circuler le mélange de gaz contenu dans cette section et à ramener ce mélange de gaz en contact avec la solution contenue dans le générateur d'agent stérilisant,

- une section de protection de sortie pourvue de moyens aptes à y créer un flux d'air stérile dirigé de manière à empêcher que le mélange gazeux contenu dans la secton précédente du tunnel (section de stérilisation) ne puisse s'échapper par la sortie du tunnel.

Dans un appareil suivant l'invention, le générateur d'agent stérilisant peut être monté à l'interieur ou à l'extérieur de la chambre (ou du tunnel).

Suivant une autre particularité d'exécution des appareils suivant l'invention, le générateur de vapeur d'eau entretient une tension de vapeur d'eau au maximum égala à la pression atmosphérique.

Suivant une autre forme de réalisation avantageuse, les moyens de chauffage et régulation thermique du générateur de gaz de stérilisation entretiennent la même température que dans le générateur de vapeur d'eau.

Les appareils suivant l'invention comportent donc des moyens aptes à introduire le mélange de gaz produit par le générateur d'agent stérilisant dans l'espace de stérilisation, à faire circuler le mélange de gaz contenu dans cet espace et à le ramener en contact avec la solution contenue dans le générateur d'agent stérilisant.

Suivant une forme d'exécution particulière, ces moyens comportent une pompe à anneau liquide dont le liquide est prélevé et ramené de manière continue dans la solution contenue dans le générateur d'agent stérlisant.

Suivant une autre forme d'exécution particulière, ces moyens comportent une trompe à liquide dont le liquide, propulsé par une pompe de circulation de liquide, est préleve et ramené de manière continue dans la solution contenue dans le générateur d'agent sterilisant.

Les appareils suivant l'invention comprennent avantageusement des moyens pour programmer es opérations du procédé.

Les formes de réalisation et particularités de fonctionnement décrites ci-dessus offrent différents avantages, notamment:

- le maintien d'une température de 60°C dans tout l'appareil évite les tensions de vapeur supérieures à la pression atmosphérique et dès lors des accessoires et mesures de sécurité que requièrent les autoclaves et chaudières et permet de simplifier les circuits de régulation thermique;
- la circulation de l'atmosphère de la chambre sous l'impulsion d'une pompe à anneau liquide aide à la régulation thermostatique par la dissipation d'énergie de la pompe dans le liquide de l'anneau;
- le préchauffage du gaz vecteur à la température du générateur de vapeur assure une humidité relative faible de ce gaz et empêche un refrodissement dans la chambre, de sorte qu'il n'y a pas de risque de condensation d'eau;
- la seconde porte éventuelle permet de construire l'appareil en forme de sas établi entre une zone septique et une zone stérile;
- la fabrication de la chambre en matière plastique à paroi unique chauffante est peu onéreuse;
- la circulation de l'atmosphère de la chambre au contact de la solution d'agent stérilisant volatil assure la constance de composition de l'atmosphère grâce au rétablissement permanent des pressions partielles d'équilibre;
- l'addition d'un excipient soluble à faible tension de vapeur à la solution d'agent stérilisant volatil permet d'agir sur los pressions partielles sans changer la température, (au moins entre certaines limites);
- le maintien du générateur de vapeur à la température de la chambre évite les condensations dans celle-ci.

Des détails et particularités de l'invention ressortent de la description ci-après, donnée à titre d'exemple non limitatif, et en se référant aux dessins annexés:

les Fig. 1 et 2 sont des représentations schématiques de deux appareils de stérilisation conformes à l'invention;

les Fig. 3, 4, 5 et 6 sont des représentations de l'évolution de la temperature, de la pression, de la concentration on formaldéhyde gazeux et de l'humidité relative au cours des cycles de stérilisation effectués respectivement dans les exemples 1, 2, 3 et 4.

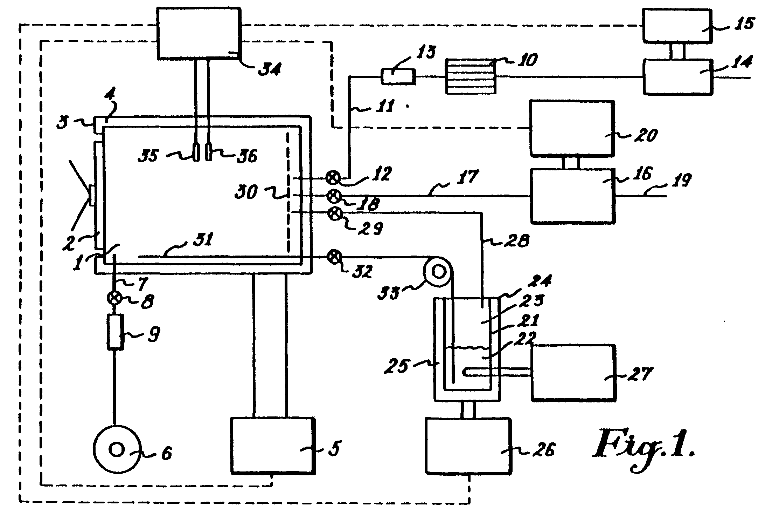

L'appareil de stérilisation représenté schématiquement à la Fig. 1 comprend une chambre de traitement 1 comportant une porte 2 pouvant se fermer hermétiquement après chargement des objets dans la chambre. La porte 2 est munie d'un moyen de chauffage, et de régulation de ce chauffage. La chambre est elle-même incluse dans une enveloppe 3 permettant la circulation d'un fluide calorifique 4, raccordée à des organes de circulation, de chauffage et de régulation des moyens de chauffage 5 à une température variable. La chambre 1 est raccordée à une pompe à vide 6 par un conduit 7 comportant une vanne 8 et un antiretour 9. La chambre 1 est raccordée à un circuit d'admission d'un gaz "vecteur" comprenant un moyen de stérilisation 10 d'un gaz, par exemple un filtre stérilisant, un conduit 11 comportant une vanne 12 et un antiretour 13. Un moyen de chauffage 14 du gaz, contrôlé par un moyen de contrôle 15, peut être monté avant le moyen de stérilisation 10. Ce moyen de chauffage 14 peut éventuellement réaliser la thermostatisation de tout 10 conduit 11 de gaz vecteur. Ce gaz peut être sous pression. La chambre 1 est raccordée à un générateur de vapeur d'eau 16 par un conduit 17 compodant une vanne 18. Le générateur 16 est raccordé à une alimentation en eau 19 et à un système de contrôle de la température et/ou de la pression 20. La chambre 1 est raccordée à un générateur d'agent stérilisant 21. Il comprend une phase liquide 22, une phase gazeuse 23 et éventuellement une phase solide. Il est inclus dans une enveloppe 24, permettant la circulation d'un fluide calorifique 25, raccordée à des moyens de circulation, de chauffage et de régulation thermique 26. Le chauffage et la température de la phase liquide 22 et éventuellement solide sont contrôlés par un moyen de contrôle et de chauffage 27. Ce système de chauffage peut faire partie du système de chauffage de la chambre 1. La phase gazeuse 23 est mise en circulation depuis le générateur d'agent stérilisant 21 vers la chambre 1 par un conduit 28 comportant une vanne 29, et répartie dans la chambre 1 par la plaque de diffusion 30. Le gaz 23 est repris par le conduit 31, comportant la vanne 32, et forcé par la pompe à gaz 33 à travers la solution 22 avant de rejoindre le conduit 28. Les conduits 28 et 31, les vannes 29 et 32 et la pompe à gaz 33 sont thermostatises à la temperature de stérilisation choisie. L'évolution de la température et de la pression est contrôlée et régulée automatiquement par le moyen de contrôle programmable 34, qui enregistre au moyen des sondes 35 et 36 la température et la pression, et commande en conséquence les vannes, la pompe à vide 6, les éléments chauffants, la pompe à gaz 33.

Lorsque l'appareil fonctionne, une solution liquide 22 éventuellement au contact avec une phase solide contenant un agent stérilisant pouvant développer une phase gazeuse 23 à la température de stérilisation choisie est introduite dans le générateur de gaz 21. Les circuits de chauffage 5, 16, 26 et 27 sont enclenchés. La charge à stériliser est introduite dans la chambre 1, et la porte 2 fermée. La chambre 1 est alors automatiquement soumise pendant un temps programmé à de la vapeur d'eau provenant du générateur de vapeur 16, dont le débit est contrôlé par la vanne 18 modulée de façon à ce que la température de la chambre 1 atteigne la température de stérilisation choisie. Une évacuation des gaz présents dans la chambre est effectuée simultanément par la pompe à vide 6. Dans une variante, un vide préalable à l'introduction de vapeur peut être effectué. Après le temps voulu, l'admission de vapeur est automatiquement coupée, alors que la pompe à vide 6 continue de fonctionner, pendant un temps préprogrammé. La pression de la chambre 1 atteint une valeur faible. La charge est à ce moment chaude et sèche. Le fonctionnement de la pompe à vide 6 est automatiquement interrompu, et la vanne 8 fermée. Les vannes 29 et 32 du générateur d'agent stérilisant 21 sont automatiquement ouvertes, et la pompe à gaz 33 mise en fonctionnement. L'atmosphère de la chambre 1 se charge ainsi progressivement en mélange de gaz stérilisant 23 provenant de l'évaporation de constituants de la phase liquide 22. Après un certain temps, la concentration du mélange de gaz stérilisant dans la chambre 1 atteint un équilibre avec la phase liquide 22 du générateur d'agent stérilisant 21 maintenu à une température constante, et s'y maintient. La pression dans la chambre 1 atteint une valeur égale à la pression régnant avant ouverture des vannes 29 et 32 du générateur d'agent stérilisant 21, additionnée à la pression due à la phase gazeuse 23 provenant de la phase liquide 22 du générateur d'agent stérilisant 21. La vanne 12 d'arrivée du gaz vecteur est alors automatiquement ouverte, et le système de chauffage 14 de ce gaz est simultanément enclenché, jusqu'à obtention d'une pression totale préprogrammée dans la chambre 1; cette pression totale peut être inférieure, égale ou supérieure à la pression atmosphérique. L'atmosphère gazeuse de la chambre 1 est alors maintenue sans changement durant la période de temps de réaction requise pour réaliser une stérilisation totale. La pompe à gaz 33 continue à fonctionner pendant ce temps et maintient l'équilibre de la phase gazeuse stérilisante 23 avec la phase liquide 22. Après le temps requis, la pompe à gaz 33 est arrêtée, les vannes 29 et 32 fermées. Un vide est alors effectué automatiquement par la pompe à vide 6, jusqu'à une pression déterminée. Le circuit de chauffage 14 du gaz vecteur est alors automatiquement enclenché et le gaz vecteur est admis dans la chambre 1 jusqu'à une pression prédéterminée. Le cycle d'évacuation et d'admission du gaz vecteur est répété un nombre suffisant de fois pour assurer l'évacuation totale du mélange de gaz stérilisant, de la chambre 1. Lors de l'admission de gaz vecteur, de la vapeur d'eau provenant du générateur de vapeur 16 par le conduit 17, peut être automatiquement admise avec le gaz vecteur. La température en est contrôlée par la sonde 35 et le système de contrôle 34. Finalement, le gaz vecteur est automatiquement admis une dernière fois dans la chambre 1, jusqu'à obtention de la pression atmosphérique. La porte 2 de la chambre 1 est ouverte, la charge est retirée, et la chambre est prête à recevoir une nouvelle charge.

Il va de soi que lorsque le générateur de vapeur 16 fonctionne à la température à laquelle la charge de stérilisation doit être portée, il devient inutile de moduler l'action de la vanne 18, d'où il résulte une simplification de régulation.

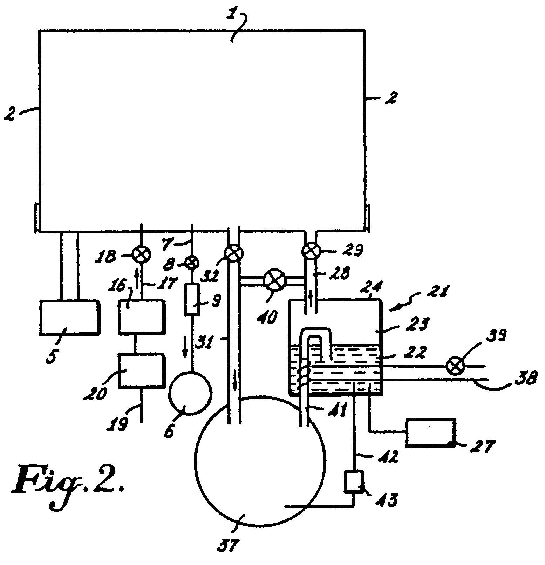

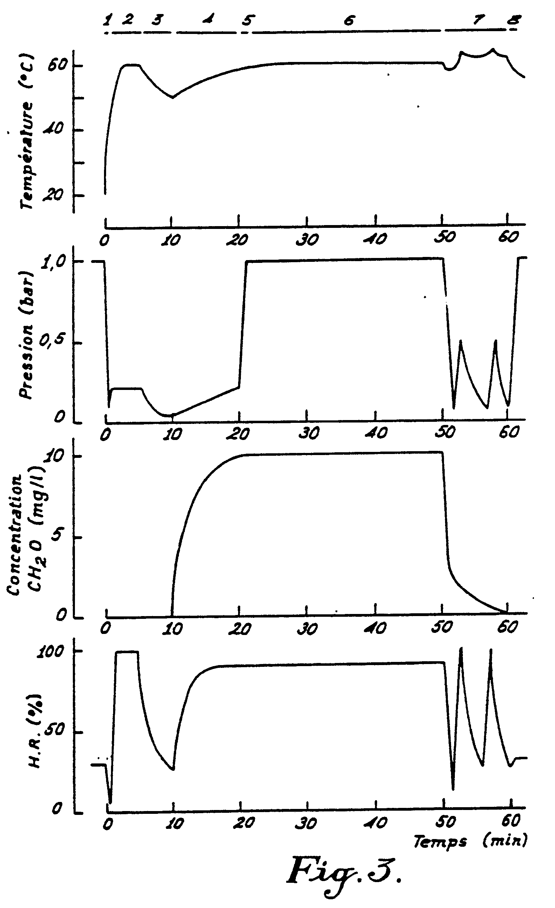

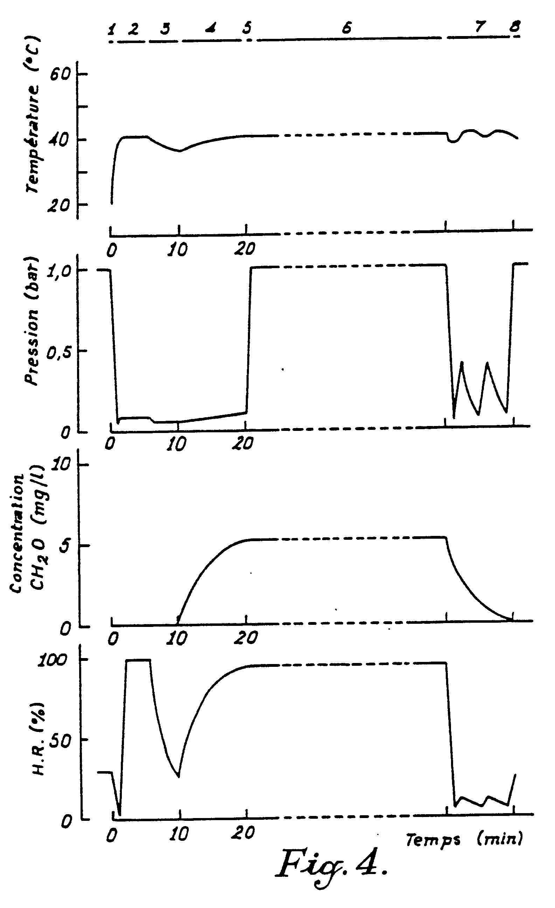

La Fig. 2 illustre un autre appareil de stérilisation, dont les éléments communs avec ceux de l'appareil de la Fig. 1 portent les mêmes références, mais ne sont représentés que lorsqu'ils interviennent dans la variante de réalisation.

L'appareil comprend une chambre de traitement 1 qui est une cuve en matière plastique comportant deux portes 2 pouvant se fermer après chargement des objets dans la chambre 1. La chambre 1 comprend des organes de chauffage (non représentés) intégrés aux parois et des moyens de régulation de chauffage 5 à une température variable. La chambre 1 est raccordée à un système à vide 6, 7, 8, 9, et à un circuit d'admission d'un gaz "vecteur" avec leurs accessoires, non représentés et semblables à ceux de la Fig. 1. La chambre 1 est raccordée à un générateur de vapeur d'eau 16 par un conduit 17 comportant une vanne 18. Le générateur 16 est raccordé à une alimentation en eau 19 et à un système de contrôle de la température et/ou de la pression 20. La chambre 1 est raccordée à un générateur d'agent stérilisant 21. Il comprend une phase liquide 22, une phase gazeuse 23 et éventuellement une phase solide. Il est inclus dans une enveloppe 24. Le chauffage et la température de la phase liquide 22 et éventuellement solide sont contrôlés par un moyen de contrôle et de chauffage 27 qui agit suivant les besoins sur le circuit de refroidissement 38 muni d'une vanne 39. La phase gazeuse 23 est mise en circulation depuis le générateur d'agent stérilisant 21 vers la chambre 1 par un conduit 28 comportant une vanne 29 sous l'impulsion d'une pompe à anneau liquide 37. Le gaz 23 est repris par le conduit 31, comportant la vanne 32. Le mélange de gaz et de liquide sortant de la pompe 37 retourne dans le réservoir 24. Le gaz séparé du liquide retourne dans la chambre 1 par le conduit 28. Un conduit de by-pass, avec vanne 40, entre les conduits 28 et 31, sert à la mise en régime de la pompe 37 ou à l'isolement de celle-ci suivant les besoins.

La pompe 37 communique par une conduite 41 coudée, qui plonge dans la solution 22, contenue dans le générateur d'agent stérilisant 21. La pompe 37 est alimentée en solution 22 par la conduite 42 munie du filtre 43.

L'appareil de la Fig. 2 fonctionne par ailleurs en substance comme celui de la Fig. 1.

La présente invention peut être appliquée à différentes compositions de liquides générateurs de gaz ou de mélanges de gaz stérilisants, à différents gaz ou mélanges de gaz vecteurs, à différentes températures et pressions maintenues durant des durées de temps variables au cours du cycle. De plus, les détails de structure, d'agencement et de disposition de l'appareil décrit (par exemple l'obtention de l'agent biocide gazeux par un barbotage en continu dans la solution) peuvent être modifiés, et un certain nombre d'éléments peuvent être remplacés par d'autres dispositifs équivalents (par exemple, les circuits de chauffage peuvent être remplacés par des résistances électriques, ou les circuits de chauffage de la chambre et du générateur de gaz peuvent être réunis en un me système).

L'invention est illustrée par les exemples non limitatifs suivants.

Dans les exemples 1, 2 et 3, on utilise pour les essais des populations connues de spores de Bacillus subtils (globigii) obtenues par culture et sporulation d'un indicateur biologique de référence (American Sterilizer Co "SPORDI" - dénomination commerciale): elles ont été déposées sur des supports de polyéthylène et séchées. Elles constituent des indicateurs biologiques (I.B. dans le texte).

Dans l'exemple 4, on utilise pour les essais des I.B. commerciaux de B. subtils (globigii) avec support en papier de marque AMSCO SPORDEX Lot 646 GBL.

Dans les quatre exemples, les populations sont soumises, emballées ou non, à un traitement destiné à les stériliser. Elles sont récupérées et mises en culture dans une solution de Tryptic Soy Broth à 30 g par litre, à 37°C pendant 14 jours.

Les exemples 1, 2 et 3 ont été effectués dans un appareil tel que représenté schématiquement à la Fig. 1, muni d'un propulseur de gaz du type ventilateur.

L'exemple 4 a été réalisé dans un appareil tel que représenté schématiquement à la Fig. 2, (avec pompe à anneau liquide).

## Exemple 1

Le générateur d'agent stérilisant 21 est rempli d'une solution à 20,7 % p/p de formaldéhyde, 5,6 % de méthanol et 73,7 % p/p d'eau. A 60°C, la phase gazeuse obtenue dans la chambre 1 est de 9,8 mg par litre de formaldéhyde gazeux et a une humidité d'environ 90 %. Un essai préalable conduit en l'absence de toute difficulté de pénétration pour le gaz, à montré que deux minutes d'exposition à ce mélange de gaz à 60°C suffisent à tuer une population de $10^6$ spores. Pour tester la capacité de stérilisation de l'invention lorsque les spores sont difficilement accessibles, les populations microbiennes et leurs supports ont été déposés dans:

- des sachets de polyéthylène (épaisseur 20 µm) soudé,
- des sachets combinés papier/plastique, du type pelable, tels qu'utilisés en stérilisation hospitalière, soudés (épaisseur du papier 60 µm épaisseur du complexe polyamide - polyéthylène 60 µm),
- des seringues de 10 ml et 2,5 ml raccordées respectivement à des tubes en P.V.C. plastifié de 3,5 m et 5 m de longueur et 2 mm de diamètre intérieur,
- des tubes en P.V.C. plastifié de 5 m de longueur et 2 mm de diamètre intérieur, raccordés par une extrémité à une capsule d'un volume interne de 1,1 ml, contenant les indicateurs biologiques (ce système est appelé "cathéter de type Helix"),
- des flacons en polyéthylène de 20 ml, munis de leurs bouchons desserrés d'un quart de tour.

Pour ces essais, le cycle suivant de stérilisation à été appliqué après thermostatisation à 60° C des moyens de chauffage 5, 14, 26 et 27:

1) un vide initial jusqu'à 0,1 bar (durée de pompage: 0,5 min),

2) un chauffage des objets à la vapeur saturée à 60°C (durée: 5 min),

3) un séchage des objets et des parois de la chambre sous vide jusqu'à obtention d'une pression résiduelle de 0,05 bar (durée: 5 min),

4) la production de la phase gazeuse de stérilisation (durée: 10 min),

5) une admission d'air jusqu'à pression atmosphérique (durée: 0,5 min),

6) la stérilisation proprement dite (durée: de 20 à 30 min),

7) une évacuation du gaz sous vide, alternée avec des admissions de vapeur et d'air (durée: 10 min),

8) une remise à pression atmosphérique (0,5 min).

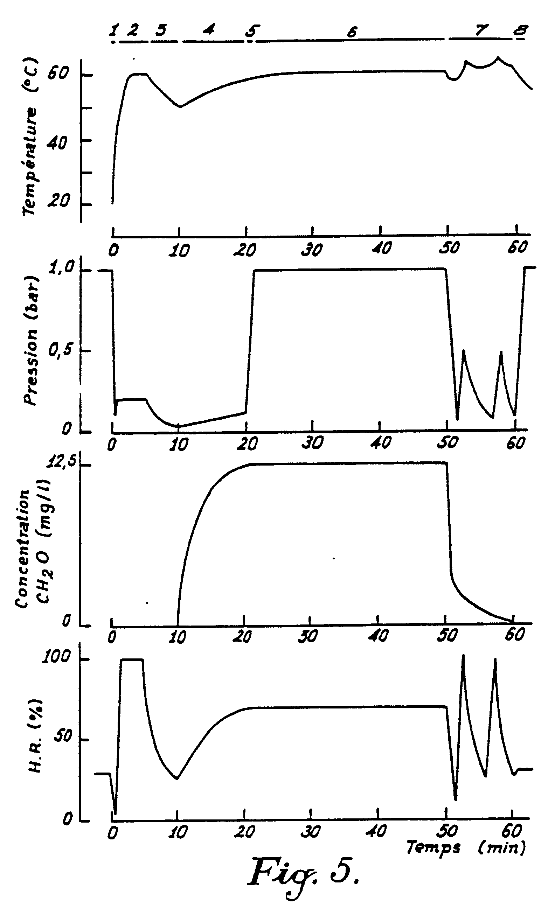

Durée totale: de 50 à 60 minutes. L'évolution de la température, de la pression totale dans la chambre, de la concentration en formaldéhyde gazeux et de l'humidité relative dans la chambre, en fonction du temps, est représentée à la Fig. 3.

Afin de vérifier si les résidus de formaldéhyde éventuellement présents dans le support de polyéthylène des indicateurs peuvent inhiber la croissance de germes survivants au traitement de stérilisation, une culture comparative de croissance de germes à été répétée 10 fois. Dans un même tube contenant le milieu de culture, un indicateur ayant subi l'autoclavage au formaldéhyde et un indicateur n'ayant subi aucun traitement au formaldéhyde ont été introduits. Une croissance bactérienne s'est manifestée immédiatement dans chacun des 10 tubes, ce qui montre que le résidu de formaldéhyde éventuellement présent après traitement est insuffisant pour inhiber la croissance de germes qui n'auraient pas été détruits par autoclavage au formaldéhyde. Les résultats des tests de stérilisation sont présentés au tableau 1.

## Exemple 2

La même phase liquide que dans l'exemple 1 est utilisée dans le générateur d'agent stérilisant 21. A 40°C, la phase gazeuse obtenue dans la chambre 1 est de 5,1 mg par litre de formaldéhyde gazeux, et a une humidité relative d'environ 95 %.

Pour tester la capacité de stérilisation de invention lorsque les spores sont difficilement accessibles, les populations microbiennes et leur support ont été déposés dans des cathéters de type "Helix" décrit dans l'exemple 1.

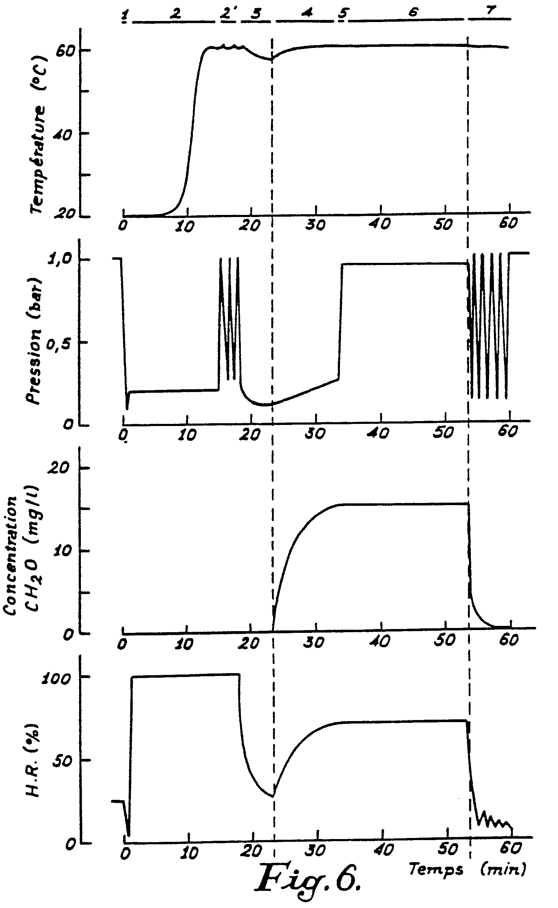

L'évolution des paramètres de température, de pression, des concentrations en formaldéhyde et d'humidité relative dans la chambre 1 est donnée à la Fig. 4. Les moyens de chauffage 5, 14, 26, 27 ont été thermostatisés à 40°C. Les résultats des tests de stérilisation sont présentés au tableau II.

### Tableau I

| Matériel contenant les indicateurs biologiques | Nombre d'I.B. et population | Temps de stérilisation (min) (étape 6) | Nombre d'I.B. stériles/ nombre total d'I.B. |
|---|---|---|---|
| Sachet polyéthylène | $8 \times 10^{5.6}$ | 30 | 16/16 |
| | $8 \times 10^{6.5}$ | 30 | |
| Sachet papier plastique thermosoudé | $4 \times 10^{6.5}$ | 30 | 43/43 |
| | $19 \times 10^{6.3}$ | 30 | |
| | $20 \times 10^{6.3}$ | | |
| Flacons polyéthylène (bouchon ouvert d'un quart de tour) | $3 \times 10^{6.5}$ | 30 | 16/16 |
| | $13 \times 10^{6.3}$ | 30 | |
| Seringue de 10 ml | $1 \times 10^{5.6}$ | 30 | |
| | $1 \times 10^{6.3}$ | 30 | 8/8 |
| | $1 \times 10^{6.3}$ | 20 | |
| | $5 \times 10^{6.2}$ | 20 | |
| Seringue de 2,5 ml | $2 \times 10^{5.6}$ | 30 | |
| | $1 \times 10^{6.3}$ | 30 | 9/9 |
| | $1 \times 10^{6.3}$ | 20 | |
| | $5 \times 10^{6.2}$ | 20 | |
| Cathéter de type "Helix" | $16 \times 10^{6.3}$ | 20 | 38/38 |
| | $22 \times 10^{6.2}$ | 20 | |

### Tableau II

| Matériel contenant les indicateurs biologiques | Nombre d'I.B. et population | Temps de stérilisation (min) | Nombre d'I.B. stériles/ nombre total d'I.B. |
|---|---|---|---|
| Cathéter de type "Helix" | $22 \times 10^{6.2}$ | 30 | 0/22 |
| | $22 \times 10^{6.2}$ | 60 | 6/22 |
| | $22 \times 10^{6.2}$ | 120 | 13/22 |
| | $22 \times 10^{6.2}$ | 240 | 22/22 |

## Exemple 3

Le générateur d'agent de stérilisation 21 est rempli d'une solution à 20,3 % p/p de formaldéhyde, 5,5 % p/p de méthanol, 29,2 % p/p d'eau et 45,1 % p/p de propylèneglycol. A 60°C, la phase gazeuse obtenue dans la chambre est de 12,1 g par litre de formaldéhyde gazeux et à une humidité relative d'environ 70 %.

Pour tester la capacité de stérilisation de l'invention lorsque les spores sont difficilement accessibles, les populations microbiennes et leurs supports ont été déposées dans des cathéters de type "Helix" décrits dans l'exemple 1. L'évolution des paramètres de température, de pression, de concentration en formaldéhyde et d'humidité dans la chambre est donnée à la Fig. 5. Les moyens de chauffage 5, 14, 26, 27 ont été thermostatisés à 60°C. Les résultats des tests de stérilisation sont les suivants:

### Tableau III

| Nombre d'I.B. et population | Temps de stérilisation (min) (étape 6) | Nombre d'I.B. stériles/nombre |
|---|---|---|
| $17 \times 10^{6.2}$ | 30 | 17/17 |

## Exemple 4

Le réservoir 24 du générateur d'agent stérilisant 21 est partiellement rempli d'une solution à 20,3 % p/p de formaldéhyde, 5,5 % p/p de méthanol, 29,2 % p/p d'eau et 45,1 % p/p de propylèneglycol, et alimente, au moyen d'une conduite, la pompe à anneau liquide 37. A 60°C, la phase gazeuse obtenue dans la chambre est de 17,35 mg par litre de formaldéhyde gazeux et a une humidité relative d'environ 70 %. Pour tester la capacité de stérilisation de invention lorsque les spores sont dans un environnement pouvant absorber significativement le formaldéhyde gazeux et la vapeur d'eau, les populations microbiennes et leur support de polyéthylène ont été déposés dans des sachets combinés papier/plastique, du type pelable, tels qu'utilisés en stérilisation hospitalière, soudés. Des indicateurs biologiques commerciaux avec support papier ont été utilisés dans leur emballage d'origine en papier (AMSCO SPORDEX Lot 646 GBL). Les indicateurs emballés ont alors été déposés au centre d'une pile de 30 linges de coton de 65 x 70 cm, pliés chacun trois fois. La pile de linges pesait 3 kg. L'ensemble à été emballé dans un papier de stérilisation, et est appelé "Test de Bowie". La température du générateur de vapeur d'eau 16 à été fixée à 60°C.

Pour ces essais, le cycle suivant de stérilisation à été appliqué après thermostatisation à 60°C des moyens de chauffage 5 et 14:

1) un vide initial jusqu'à 0,1 bar (durée de pompage: 0,5 min);

2) un chauffage des objets à la vapeur d'eau saturée jusqu'à obtention d'une température de 60°C au centre de la pile de linges (durée: environ 15 min);

2') une admission d'air jusqu'à pression atmosphérique (durée: 0,5 min), une évacuation de l'atmosphère de la chambre jusqu'à 0,3 bar (durée: 0,5 min). Ceci est répété au total 3 fois, la vanne 18 restant ouverte;

3) un séchage de la charge et des parois sous vide jusqu'à obtention d'une pression résiduelle de 0,1 bar, la vanne 18 étant fermée (durée: 5 min), ceci achevant le préconditionnement;

4) la production de la phase gazeuse de stérilisation (durée 10 min);

5) une admission d'air jusqu'à obtention d'une pression de 0,95 bar (durée: 0,5 min) avec arrêt de la pompe à gaz 37;

6) la stérilisation proprement dite (durée: 20 min), ceci complétant la phase de stérilisation;

7) une évacuation du gaz sous vide, la vanne 18 étant ouverte au moins une fois si nécessaire, alternée avec des admissions d'air (durée: 5 min);

8) une remise à pression atmosphérique (durée: 0,5 min), ceci achevant le postconditionnement (durée totale: 60 min).

L'évolution de la température, de la pression totale dans la chambre, de la concentration en formaldéhyde gazeux et de l'humidité relative dans la chambre, en fonction du temps, est représentée à la Fig. 6.

Les résultats des tests de stérilisation sont présentés au tableau IV.

### Tableau IV

| Type des indicateurs biologiques | Nombre d'I.B. et population | Temps de stérilisation (min) (étape 6) | Nombre d'I.B. stériles/ nombre total d'I.B. |
|---|---|---|---|
| Polyéthylène | $35 \times 10^{5.9}$ | 20 | 35/35 |
| Papier | $13 \times 10^{5.7}$ | 20 | 13/13 |

## Revendications

1. Procédé de stérilisation d'une matière ou d'un objet par la mise en contact de cette matière ou de cet objet avec un mélange de gaz contenant au moins un agent stérilisant obtenu par évaporation d'une solution liquide (22), caractérisé en ce que la matière ou l'objet à stériliser et le mélange de gaz contenant au moins un agent stérilisant sont confinés dans une chambre (1) étanche aux fluides, le mélange de gaz contenant au moins un agent stérilisant est propulsé en circuit et ramené en contact avec la solution liquide (22) qui contient l'agent stérilisant à l'état dissous, liquide et/ou solide, en concentration suffisante pour établir la concentration désirée en agent stérilisant évaporé, et le mélange de gaz confiné dans la chambre (1) est soumis à des variations de pression qui sont supérieures aux seules différences de pression nécessaires à la propulsion en circuit du mélange de gaz contenant au moins un agent stérilisant.

2. Procédé suivant la revendication 1, caractérisé en ce que l'agent stérilisant est le formaldéhyde.

3. Procédé suivant la revendication 2, caractérisé en ce que la solution (22) est une solution aqueuse de formaldéhyde, contenant éventuellement d'autres composants tels que des sels minéraux ou organiques, des polyols comme la glycérine, le propylèneglycol, l'éthylèneglycol ou des polyoxyéthylèneglycols.

4. Procédé suivant l'une quelconque des revendications 1 à 3, caractérisé en ce que la température du mélange de gaz contenant au moins un agent stérilisant se situe entre 20 et 100°C et que la matière ou l'objet à stériliser sont portés, avant la mise en contact avec le mélange de gaz contenant au moins un agent stérilisant, à une température proche de celle de ce mélange de gaz ou égale à celle-ci.

5. Procédé suivant la revendication 4, caractérisé en ce que la température du mélange de gaz contenant au moins un agent stérilisant se situe entre 55 et 65°C.

6. Procédé suivant l'une quelconque des revendications 1 à 5, caractérisé en ce qu'une part au moins des parties de la chambre (1) en contact avec le mélange de gaz contenant au moins un agent stérilisant à été portée à la température du mélange de gaz contenant au moins un agent stérilisant.

7. Procédé suivant l'une quelconque des revendications 1 à 6, caractérisé en ce qu'au cours de son exécution, le mélange de gaz confiné dans la chambre (1) est soumis une ou plusieurs fois à une augmentation de pression, par introduction dans la chambre (1) d'un gaz ou d'un mélange de gaz vecteur.

8. Procédé suivant la revendication 7, caractérisé en ce que le gaz ou mélange de gaz vecteur est l'azote ou l'air ou un mélange de ceux-ci.

9. Procédé suivant l'une quelconque des revendications 7 et 8 caractérisé en ce que:
- on introduit la matière ou l'objet à stériliser dans la chambre (1),
- on amène cette chambre (1) à une première pression subatmosphérique,
- on introduit de la vapeur d'eau à une température égale ou voisine de celle à laquelle on veut porter la matière ou l'objet, jusqu'à obtention de la température voulue, une succion partielle hors de la chambre (1) de cette vapeur et de l'eau condensée étant continuellement effectuée,
- on interrompt l'admission de vapeur d'eau,
- on amène la chambre (1) à une seconde pression subatmosphérique jusqu'à disparition de l'eau qui s'est condensée lors de l'introduction de vapeur d'eau,
- on met la chambre (1) en communication avec la solution (22) maintenue à une température constante, égale à la température choisie pour le mélange de gaz qui est obtenu par évaporation de cette solution (22),
- on fait circuler le mélange de gaz contenu dans la chambre en le ramenant de manière continue en contact avec la solution (22),
- après que la teneur en agent stérilisant du mélange de gaz contenu dans la chambre (1) s'est mise en équilibre avec la solution (22), on introduit dans la chambre (1) un gaz ou un mélange de gaz vecteur, en y augmentant ainsi la pression jusqu'à obtention d'une pression totale inférieure, égale ou supérieure à la pression atmosphérique,
- on expose ainsi la surface de la matière ou de l'objet pendant le temps requis pour obtenir la stérilité, tandis que le mélange de gaz contenu dans la chambre (1) est maintenu en circulation et ramené en contact de manière continue avec la solution (22),
- on coupe la communication entre la solution (22) et le mélange de gaz contenu dans la chambre (1),
- on élimine l'agent stérilisant contenu dans la chambre (1),
- on ramène l'intérieur de la chambre (1) à la pression atmosphérique, et
- on en retire la matière ou l'objet.

10. Procédé suivant l'une quelconque des revendications 7 et 8 caratérisé en ce que:
- on introduit la matière ou l'objet à stériliser dans la chambre (1),
- on amène cette chambre (1) à une première pression subatmosphérique,
- on introduit de la vapeur d'eau à une température égale ou voisine de celle à laquelle on veut porter la matière ou l'objet, jusqu'à obtention de la température voulue une succion partielle hors de la chambre (1) de cette vapeur et de l'eau condensée étant continuellement effectuée,
- l'introduction de la vapeur d'eau restant possible, on introduit une ou plusieurs fois dans la chambre (1) un gaz ou mélange de gaz vecteur, jusqu'à obtention d'une pression totale inférieure, égale ou supérieure à la pression atmosphérique et après chaque introduction de gaz ou mélange de gaz vecteur, on ramène la chambre (1) à une pression subatmosphérique inférieure à celle qu'on y a obtenue par l'introduction du gaz en mélange de gaz vecteur,
- on interrompt l'admission de vapeur d'eau,
- on amene la chambre (1) à une seconde pression subatmosphérique jusqu'à disparition de l'eau qui s'est condensée lors de l'introduction de vapeur d'eau,
- on met la chambre (1) en communication avec la solution (22) maintenue à une température constante, égale à la température choisie pour le mélange de gaz qui est obtenu par évaporation de cette solution (22),
- on fait circuler le mélange de gaz contenu dans la chambre en le ramenant de manière continue en contact avec la solution (22),
- après que la teneur en agent stérilisant du mélange de gaz contenu dans la chambre (1) s'est mise en équilibre avec la solution (22), on introduit dans la chambre (1) un gaz ou un mélange de gaz vecteur, en y augmentant ainsi la pression jusqu'à obtention d'une pression totale inférieure, égale ou supérieure à la pression atmosphérique,
- on expose ainsi la surface de la matière ou de l'objet pendant le temps requis pour obtenir la stérilité, tandis que le mélange de gaz contenu dans la chambre (1) est maintenu en circulation et ramené en contact de manière continue avec la solution,
- on coupe la communication entre la solution (22) et le mélange de gaz contenu dans la chambre (1),
- on élimine l'agent stérilisant contenu dans la chambre (1),
- on ramène l'intérieur de la chambre (1) à la pression atmosphérique, et
- on en retire la matière ou l'objet.

11. Appareil pour la stérilisation de matières ou d'objets par mise en contact avec un mélange de gaz contenant au moins un agent stérilisant obtenu par évaporation d'une solution liquide (22), cet appareil comprenant une chambre de traitement (1) étanche aux fluides, à régulation thermostatique (3, 4, 5) munie d'au moins une porte conçue pour charger des matières ou objets dans la chambre (1) et les en retirer, caractérisé en ce qu'il comprend en outre:

- un générateur d'agent stérilisant (21) apte à contenir une solution (22) de cet agent stérilisant et à évaporer cette solution (22) à une température predéterminée, grâce à des moyens de chauffage et/ou refroidissement et des moyens de régulation thermique (24, 25, 26, 27),

- des moyens (33) aptes à introduire le mélange de gaz produit par le générateur d'agent stérilisant (21) dans la chambre (1), à faire circuler le mélange de gaz contenu dans la chambre (1) et à le ramener en contact avec la solution (22) contenue dans le générateur d'agent stérilisant (21),

- un générateur de vide (6, 7, 8, 9) raccordé à la chambre (1),

- un circuit d'admission de gaz vecteur (10, 11, 13, 14) raccorde à la chambre (1) et comprenant un moyen de stérilisation (10) d'un gaz et un moyen de chauffage thermostatique (14) de ce gaz,

- un générateur de vapeur d'eau (16) qui est raccordé d'une part à la chambre (1) et d'autre part à une alimentation en eau (19) et qui est pourvu d'un système de régulation de la température et/ou de la pression (20),

- des moyens (8, 12, 18, 29, 32) aptes à mettre le générateur d'agent stérilisant (21), le générateur de vide (6, 7 8, 9), le circuit d'admission de gaz vecteur (10, 11, 13, 14) et le générateur de vapeur d'eau (16) en communication avec la chambre (1) ou à couper ces communications.

12. Appareil suivant la revendication 11, caractérisé en ce que la chambre (1) est incluse dans une enveloppe (3) propre à la circulation d'un fluide calorifique (4) et raccordée à des moyens (5) aptes à assurer la circulation, le chauffage et la régulation du chauffage du fluide calorifique (4).

13. Appareil suivant la revendications 11, caractérisé en ce que la chambre (1) est une chambre en matière plastique non conductrice de l'électricité qui résiste aux efforts dûs aux pressions subatmospheriques et qui est munie d'organes de chauffage électriques intérieurs et/ou noyés dans la masse des parois.

14. Appareil suivant l'une quelconque des revendications 11 à 13, caractérisé en ce que le moyen de chauffage thermostatique (14) du gaz dans le circuit d'admission de gaz vecteur (10, 11, 13, 14) est un échangeur de chaleur chauffé par le générateur de vapeur d'eau (16).

15. Appareil suivant l'une quelconque des revendications 11 à 14, caractérisé en ce que la chambre (1) est munie d'une sonde thermique (35) raccordée à un dispositif de régulation qui fait persister le fonctionnement du générateur de vapeur d'eau au moins jusqu'à acquisition par la charge d'une température prédéterminée.

16. Appareil pour la stérilisation de matières ou d'objets par mise en contact avec un mélange de gaz contenant au moins un agent stérilisant obtenu par évaporation d'une solution liquide (22), caractérisé en ce qu'il consiste en un tunnel à régulation thermostatique qui est pourvu d'un transporteur apte à y faire défiler les matières ou objets et qui comprend, depuis son entrée jusqu'à sa sortie:

- une section de préchauffage pourvue de moyens aptes à porter les matières ou objets à une température prédéterminée,

- une section de protection d'entrée pourvue de moyens aptes à y créer un flux d'air préchauffé à une température prédéterminée, dirigée de manière à empêcher que le mélange gazeux contenu dans la section suivante du tunnel (section de stérilisation) ne puisse s'échapper par l'entrée du tunnel,

- une section de stérilisation qui est équipée d'un générateur d'agent stérilisant (21) apte à contenir une solution (22) de cet agent stérilisant et à évaporer cette solution (22) à une température prédéterminée, grâce à des moyens de chauffage et/ou de refroidissement et des moyens de régulation thermique (24, 25, 26, 27), et qui est également équipée de moyens (33) aptes à introduire le mélange de gaz produit par le générateur d'agent stérilisant (21) dans la section de stérilisation du tunnel, à faire circuler le mélange de gaz contenu dans cette section et à ramener ce mélange de gaz en contact avec la solution (2) contenue dans le générateur d'agent stérilisant (21),

- une section de protection de sortie pourvue de moyens aptes à y créer un flux d'air stérile dirigé de manière à empêcher que le mélange gazeux contenu dans la section précédente du tunnel (section de stérilisation) ne puisse s'échapper par la sortie du tunnel.

17. Appareil suivant l'une quelconque des revendications 11 à 16, caractérisé en ce que le générateur d'agent stérilisant (21) est à l'extérieur de la chambre (1) ou du tunnel.

18. Appareil suivant l'une quelconque des revendications 11 a 16, caractérisé en ce que le générateur d'agent stérilisant (21) est à l'intérieur de la chambre ou du tunnel.

19. Appareil suivant l'une quelconque des revendications 11 à 18, caractérisé en ce que le générateur de vapeur d'eau (16) entretient une tension de vapeur d'eau au maximum égale à la pression atmosphérique.

20. Appareil suivant l'une quelconque des revendications 11 à 19, caractérisé en ce que les moyens de chauffage et régulation thermique (24, 25, 26, 27) du générateur d'agent stérilisant (21) entretiennent la même température que dans le générateur de vapeur d'eau (16).

21. Appareil suivant l'une quelconque des revendications 11 à 20 caractérisé en ce que les moyens (33, 37) aptes à introduire le mélange de gaz (23) produit par le générateur d'agent stérilisant (21) dans l'espace de stérilisation, à faire circuler le mélange de gaz contenu dans cet espace et à le ramener en contact avec la solution (22) contenue dans le générateur d'agent stérilisant (21), comportent une pompe à anneau liquide (37), dont le liquide est prélevé et ramené de manière continue dans la solution (22) contenue dans le générateur d'agent stérilisant (21).

22. Appareil suivant l'une quelconque des revendications 11 à 20, caractérisé en ce que les moyens (33, 37) aptes à introduire le mélange de gaz (23) produit par le générateur d'agent stérilisant (21) dans l'espace de stérilisation, à faire circuler le mélange de gaz contenu dans cet espace et à le ramener en contact avec la solution (22) contenue dans le générateur d'agent stérilisant (21), comportent une trompe à liquide dont le liquide, propulsé par une pompe de circulation de liquide, est prélevé et ramené de manière continue dans la solution (22) contenue dans le générateur d'agent stérilisant (21).

23. Appareil suivant l'une quelconque des revendications 11 à 22, caractérisé en ce qu'il comprend en outre des moyens pour programmer les opérations du procédé.

**Patentansprüche**

1. Verfahren zur Sterilisation eines Materials oder eines Gegenstandes durch Inberührungbringen diese Materials oder dieses Gegenstandes mit einer mindestens ein Sterilisationmittel enthaltenden Gasmischung, die durch Verdampfen einer flüssigen Lösung (22) erhalten wird, dadurch gekennzeichnet, dass das zu sterilisierende Material oder der zu sterilisierende Gegenstand und die mindestens ein Sterilisationsmittel enthaltende Gasmischung von einer gas- und flüssigkeitsdichten Kammer (1) umschlossen sind, die mindestens ein Sterilisationsmittel enthaltende Gasmischung im Kreislauf geführt und mit der flüssigen Lösung (22) welche das Sterilisationsmittel in gelöstem, flüssigem und/oder festem zustand in ausreichender Konzentration enthält, um die gewünschte Konzentration an verdampftem Sterilisationsmittel zu gewährleisten, wieder in Berührung gebracht wird, und die von der Kammer (1) umschlossene Gasmischung Druckänderungen unterworfen wird, welche über den blossen Druckunterschieden liegen, welche zur Führung der mindestens ein Sterilisationsmittel enthaltenden Gasmischung im Kreislauf notwendig sind.

2. Verfahren nach Anspruch 1, dadurch gekennzeichnet, dass das Sterilisationsmittel Formaldehyd ist.

3. Verfahren nach Anspruch 2, dadurch gekennzeichnet, dass die Lösung (22) eine wässerige Formaldehydlösung ist, welche gegebenfalls andere Bestandteile wie Mineralsalze oder organische Salze, Polyole wie Glycerin, Propylenglycol, Äthylenglycol oder Polyoxyäthylenglycole enthält.

4. Verfahren nach einem der Ansprüche 1 bis 3, dadurch gekennzeichnet, dass die Temperatur der mindestens ein Sterilisationsmittel enthaltenden Gasmischung im Bereich zwischen 20 und 100°C liegt und das zu sterilisierende Material oder der zu sterilisierende Gegenstand vor Inberührungbringen mit der mindestens ein Sterilisationsmittel enthaltenden Gasmischung auf eine Temperatur nahe oder gleich jener der Gasmischung gebracht wird.

5. Verfahren nach Anspruch 4, dadurch gekennzeichnet, dass die Temperatur der mindestens ein Sterilisationsmittel enthaltenden Gasmischung im Bereich zwischen 55 und 65°C liegt.

6. Verfahren nach einem der Ansprüche 1 bis 5, dadurch gekennzeichnet, dass mindestens ein Teil der Kammer (1), der mit der mindestens ein Sterilisationsmittel enthaltenden Gasmischung in Berührung steht, auf die Temperatur der mindestens ein Sterilisationsmittel enthaltenden Gasmischung gebracht wurde.

7. Verfahren nach einem der Ansprüche 1 bis 5, dadurch gekennzeichnet, dass während seiner Durchführung die von der Kammer (1) umschlossene Gasmischung durch Einleitung eines Traggases oder einer Traggasmischung in die Kammer (1) ein oder mehrere Male einer Druckerhöhung unterworfen wird.

8. Verfahren nach Anspruch 7, dadurch gekennzeichnet, dass das Traggas oder die Traggasmischung Stickstoff oder Luft oder eine Mischung derselben ist.

9. Verfahren nach einem der Ansprüche 7 und 8, dadurch gekennzeichnet, dass:
- man in die Kammer (1) das zu sterilisierende Material oder den zu sterilisierenden Gegenstand einbringt;
- man diese Kammer (1) auf einen ersten Unteratmosphärendruck bringt;
- man Wasserdampf mit einer Temperatur gleich oder nahe jener, auf die man das Material oder den Gegenstand bringen will, bis zum Erhalt der gewünschten Temperatur einleitet, wobei ein teilweises Absaugen dieses Dampfes une des kondensierten Wassers ausserhalb der Kammer (1) kontinuierlich erfolgt;
- man die zufuhr von Wasserdampf unterbricht;
- man die Kammer (1) auf einen zweiten Unteratmosphärendruck bringt bis zum Verschwinden des Wassers, welches während der Einleitung von Wasserdampf kondensierte;
- man die Kammer (1) mit der Lösung (22) in Verbindung bringt, welche auf einer konstanten Temperatur gehalten wird, welche gleich der gewählten Temperatur für die Gasmischung ist, die durch Verdampfen dieser Lösung (22) erhalten wird;
- man die in der Kammer enthaltene Gasmischung in einer kontinuierlichen zurückführenden Weise zwecks Inberührungbringen mit der Lösung (22) zirkulieren lässt;
- nach dem sich bezüglich des Gehaltes an Sterilisationsmittel der in der Kammer (1) enthaltenen Gasmischung ein Gleichgewicht mit des Lösung (22) eingestellt hat, man in die Kammer (22) ein Traggas oder eine Traggasmischung einleitet, um auf diese Weise den Druck zu erhöhen bis ein Gesamtdruck erhalten wird, der unter, gleich oder über dem Atmosphärendruck ist;

- man so die oberfläche des Materials oder Gegenstandes während der erfoderlichen zeit der Sterilisation aussetzt, um Sterilität zu erhalten, wobei die in der Kammer (1) enthaltenen Gasmischung in Zirkulation gehalten und in kontinuierlichen Weise zwecks Inberührungbringen mit der Lösung (22) zurückgeführt wird;

- man die Verbindung zwischen der Lösung (22) und der in der Kammer (1) enthaltenen Gasmischung unterbricht;

- man das in der Kammer (1) enthaltene Sterilisationsmittel entfernt;

- man das Innere der Kammer (1) auf Atmosphärendruck zurückführt, und

- man das Material oder den Gegenstand herausnimmt.

10. Verfahren nach einem der Ansprüche 7 und 8, dadurch gekennzeichnet, dass:

- man in die Kammer (1) das zu sterilisierende Material oder den zu sterilisierenden Gegenstand einbringt;

- man diese Kammer (1) auf einen ersten Unteratmosphärendruck bringt;

- man Wasserdampf mit einer Temperatur gleich oder nahe jener, auf die man das Material oder den Gegenstand bringen will, bis zum Erhalt der gewünschten Temperatur einleitet, wobei ein teilweises Absaugen dieses Dampfes und des kondensierten Wassers ausserhalb der Kammer (1) kontinuierlich erfolgt;

- bei möglich bleibender Einleitung von Wasserdampf man in die Kammer (1) ein oder mehrere Male ein Traggas oder eine Traggasmischung bis zur Erreichung eines Gesamtdruckes einleitet, der unter, gleich oder über dem Atmosphärendruck ist, und nach jeder Einleitung von Traggas oder Traggasmischung man die Kammer (1) auf einen Unteratmosphärendruck zurückführt, der unter jenem liegt, der durch Einleitung des Traggases oder der Traggasmischung erhalten wird;

- man die zufuhr von Wasserdampf unterbricht;

- man die Kammer (1) auf einen zweiten Unteratmosphärendruck bis zum Verschwinden des Wassers bringt, welches während der Einleitung von Wasserdampf kondensierte;

- man die Kammer (1) mit der Lösung (22) in Verbindung bringt, welche auf einer konstanten Temperatur gehalten wird, welche gleich der gewählten Temperatur für die Gasmischung ist, die durch Verdampfen dieser Lösung (22) erhalten wird;

- man die in der Kammer enthaltene Gasmischung in einer kontinuierlichen zurückführenden Weise zwecks Inberührungbringen mit der Lösung (22) zirkulieren lässt;

- nachdem sich bezüglich des Gehaltes an Sterilisationsmittel der in der Kammer (1) enthaltenen Gasmischung ein Gleichgewicht mit des Lösung (22) eingestellt hat, man in die Kammer (1) ein Traggas oder eine Traggasmischung einleitet, um auf diese Weise den Druck zu erhöhen bis ein Gesamtdruck erhalten wird, der unter, gleich oder über dem Atmosphärendruck ist;

- man so die Oberfläche des Materials oder Gegenstandes während der erfoderlichen Zeit der Sterilisation aussetzt, um Sterilität zu erhalten, wobei die in der Kammer (1) enthaltene Gasmischung in Zirkulation gehalten und in kontinuierlicher Weise zwecks Inberührungbringen mit der Lösung (22) zurückgeführt wird;

- man die Verbindung zwischen der Lösung (22) und der in der Kammer (1) enthaltenen Gasmischung unterbricht;

- man das in der Kammer (1) enthaltene Sterilisationsmittel entfernt;

- man das Innere der Kammer (1) auf Atmosphärendruck zurückführt, und

- man das Material oder den Gegenstand herausnimmt.

11. Vorrichtung zur Sterilisation von Materialien oder Gegenständen Inberührungbringen mit einer Gasmischung, die mindestens ein Sterilisationsmittel enthält und durch Verdampfen einer flüssigen Lösung (22) erhalten wird, wobei diese Vorrichtung eine mediumsdichte Behandlungskammer (1) mit thermostatischer Steuerung (3, 4, 5) aufweist, welche mit mindestens einer Tür zum Beschicken der Kammer (1) mit Materialien oder Gegenständen und zum Herausnehmen derselben versehen ist, dadurch gekennzeichnet, dass sie ausserdem umfasst:

- einen Entwickler (21) für Sterilisationsmittel, der zur Aufnahme einer Lösung (22) dieses Sterilisationsmittel und zur Verdampfung dieser Lösung (22) bei einer vorbestimmten Temperatur mittels Heiz- und/oder Kühleinrichtungen und thermischer Steuereinrichtungen (24, 25, 28, 27) geeignet ist;

- Einrichtungen (33) zum Einleiten der durch den Entwickler (21) für das Sterilisationsmittel erzeugten Gasmischung in die Kammer (1), zum Zirkulierenlassen der in der Kammer (1) enthaltenen Gasmischung und zum Zurückführen zwecks Inberührungbringen mit Lösung (22), welche in dem Entwickler (21) für das Sterilisationsmittel enthalten ist;

- einen Vakuumerzeuger (8, 7, 8, 9), der mit der Kammer (1) verbunden ist;

- eine Zufuhrleitung (10, 11, 13, 14) für Traggas, die mit der Kammer (1) verbunden ist und eine Einrichtung (10) zur Sterilisation eines Gases und eine thermostatische Heizeinrichtung (14) für dieses Gas enthält;

- einen Wasserdampferzeuger (18), welcher einerseits mit der Kammer (1) und andererseits mit einer Wasserspeisungsstelle (19) verbunden ist und welcher mit einem System (20) zur Steuerung der Temperatur und/oder des Druckes versehen ist;

- Einrichtungen (8, 12, 18, 29, 32), um den Entwickler (21) für das Sterilisationsmittel, den Vakuumerzeuger (8, 7, 8, 9), die Leitung (10, 11, 13, 14) zur zufuhr von Traggas und den Wasserdampferzeuger (16) mit der Kammer (1) zu verbinden oder diese Verbindungen zu unterbrechen.

12. Vorrichtung nach Anspruch 11, dadurch gekennzeichnet, dass die Kammer (1) in einer Umhüllung (3) eingeschlossen ist, die für die Zirkulation eines Wärmemediums (4) geeignet und mit Einrichtungen (5) zur Sicherung der Zirkulation, der Erhitzung und der Heizungsregulierung des Wärmemediums (4) verbunden ist.

13. Vorrichtung nach Anspruch 11, dadurch gekennzeichnet, dass die Kammer (1) eine Kammer aus elektrisch nichtleitendem Kunststoff ist, welche den durch die unteratmosphärischen Drücken bewirkten Beanspruchungen widersteht und welche innen oder eingebettet in die Masse der Wände mit elektrischen Heizorganen versehen ist.

14. Vorrichtung nach einem der Ansprüche 11 bis 13, dadurch gekennzeichnet, dass die thermostatische Heizeinrichtung (14) fur das Gas in der Zufuhrleitung (10, 11, 13, 14) des Traggases ein Wärmeaustauscher ist, der durch den Wasserdampferzeuger (16) erhitzt wird.

15. Vorrichtung nach einem der Ansprüche 11 bis 14, dadurch gekennzeichnet, dass die Kammer (1) mit einer Wärmesonde (35) versehen ist, die mit einer Steuereinrichtung verbunden ist, welche den Wasserdampferzeuger mindestens so lange in Betrieb hält, bis die Charge eine vorbestimmte Temperatur angenommen hat.

16. Vorrichtung zur Sterilisation von Materialien oder Gegenständen durch Inberührungbringen mit einer mindestens ein Sterilisationsmittel enthaltenden Gasmischung, die durch Verdampfen einer flüssigen Lösung (22) erhalten wird, dadurch gekennzeichnet, dass sie aus einem Tunnel mit thermostatischer Regelung besteht, welcher mit einem Förderer zum Transportieren der Materialien oder Gegenstände ausgestattet ist und welcher zwischen seinem Eingang und seinem Ausgang umfasst;

- einen Abschnitt zur Vorerhitzung, der mit Einrichtungen versehen ist, um die Materialien oder Gegenstände auf eine vorbestimmte Temperatur zu bringen;

- einen Abschnitt zum Eintrittschutz, der mit Einrichtungen versehen ist, um einem Strom vorerhitzter Luft mit einer vorbestimmten Temperatur zu erzeugen, der so geleitet ist, dass die in dem nachfolgenden Abschnitt des Tunnels (Sterilisationsabschnitt) enthaltene Gasmischung nicht durch den Tunneleingang entweichen kann;

- einem Sterilisationsabschnitt, welcher mit einem Entwickler (21) für das Sterilisationsmittel ausgestattet ist, der geeignet ist eine Lösung (22) dieses Sterilisationsmittel aufzunehmen und diese Lösung (22) bei einer vorbestimmten Temperatur mittels Heiz- und/oder Kühleinrichtungen und thermischer Regulierungseinrichtungen (24, 25, 26, 27) zu verdampfen, und welcher auch mit Einrichtungen (33) ausgestattet ist, welche geeignet sind, die durch den Entwickler (21) für das Sterilisationsmittel erzeugte Gasmischung in den Sterilisationsabschnitt des Tunnels einzuleiten, die in diesem Abschnitt enthaltene Gasmischung zirkulieren zu lassen und diese Gasmischung zum Inberührungbringen mit der in dem Entwickler (21) für das Sterilisationsmittel enthaltenen Lösung (22) zurückzuführen;

- einen Abschnitt zum Austrittschutz, der mit Einrichtungen versehen ist, die geeignet sind, einen Strom steriler Luft zu erzeugen, der so geleitet ist, dass die im vorhergehenden Abschnitt des Tunnels (Sterilisationsabschnitt) enthaltene Gasmischung nicht durch den Tunnelausgang entweichen kann.

17. Vorrichtung nach einem der Ansprüche 11 bis 16, dadurch gekennzeichnet, dass der Entwickler (21) für das Sterilisationsmittel ausserhalb der Kammer (1) oder des Tunnels vorgesehen ist.

18. Vorrichtung nach einem der Ansprüche 11 bis 16, dadurch gekennzeichnet, dass der Entwickler (21) für das Sterilisationsmittel innerhalb der Kammer oder des Tunnels vorgesehen ist.

19. Vorrichtung nach einem der Ansprüche 11 bis 18, dadurch gekennzeichnet, dass der Wasserdampferzeuger (16) einen Wasserdampfdruck liefert, der maximal gleich dem Atmosphärendruck ist.

20. Vorrichtung nach einem der Ansprüche 11 bis 19, dadurch gekennzeichnet, dass die Heizeinrichtung und thermische Steuerung (24, 25, 26, 27) des Entwicklers (21) für das Sterilisationsmittel dieselbe Temperatur liefert wie der Wasserdampferzeuger (16).

21. Vorrichtung nach einem der Ansprüche 11 bis 20, dadurch gekennzeichnet, dass die Einrichtungen (33, 37) zum Einleiten der durch den Entwickler (21) für das Sterilisationsmittel erzeugten Gasmischung (23) in der Sterilisationsraum, zum Zirkulierenlassen der in diesem Raum enthaltenen Gasmischung und zum Zurückführen zwecks Inberührungbringen mit der Lösung (22), welche in dem Entwickler (21) für das Sterilisationsmittel enthalten ist, eine Flüssigkeitsringpumpe (37) aufweist, deren Flüssigkeit kontinuierlich der in dem Entwickler (21) für das Sterilisationsmittel enthaltenen Lösung (22) entnommen und wieder zugeführt wird.

22. Vorrichtung nach einem der Ansprüche 11 bis 20, dadurch gekennzeichnet, dass die Einrichtungen (33, 37) zum Einleiten der durch den Entwickler (21) für das Sterilisationsmittel erzeugten Gasmischung (23) in den Sterilisationsraum, zum Zirkulierenlassen der in diesem Raum enthaltenen Gasmischung und zum Zurückführen zwecks Inberührungbringen mit der Lösung (22), welche in dem Entwickler (21) für das Sterilisationsmittel enthalten ist, eine Flüssigkeitsstrahlpumpe aufweist, deren Flüssigkeit, bewegt durch eine Flüssigkeitsumlaufpumpe, kontinuierlich der in dem Entwickler (21) für das Sterilisationsmittel enthaltenen Lösung (22) entnommen und wieder zugeführt wird.

23. Vorrichtung nach einem der Ansprüche 11 bis 22, dadurch gekennzeichnet, dass sie ausserdem Einrichtungen zum Programmieren der Arbeitsgänge des Verfahrens aufweist.

**Claims**

1. A process for sterilising a material or an article by placing this material or this article in contact with a gas mixture containing at least one sterilising agent produced by vaporising a liquid solution (22), characterised in that the material or article to be sterilised and the gas mixture containing at least one sterilising agent are enclosed in a fluid-tight chamber (1), the gas mixture containing at least one sterilising agent is propelled in a circuit and brought into contact with the liquid solution (22) which contains the sterilising agent in a dissolved, liquid and/or solid form, in a concentration which is sufficient to produce the desired concentration of the vaporised sterilising agent, and the gas mixture enclosed in the chamber (1) is subjected to pressure changes which are greater than the only pressure changes required for propelling the gas mixture containing at least one sterilising agent in a circuit.

2. A process according to Claim 1, characterised in that the sterilising agent is formaldehyde.

3. A process according to Claim 2, characterised in that the solution (22) is an aqueous solution of formaldehyde, if appropriate containing other components such as inorganic or organic salts, polyols such as glycerine, propylene glycol or polyoxyethylene glycols.

4. A process according to any one of Claims 1 to 3, characterised in that the temperature of the gas mixture containing at least one sterilising agent is between 20 and 100°C and the material or article to be sterilised is brought, before being placed in contact with the gas mixture containing at least one sterilising agent, to a temperature close to that of this gas mixture or equal to it.

5. A process according to Claim 4, characterised in that the temperature of the gas mixture containing at least one sterilising agent is between 55 and 65°C.

6. A process according to any one of Claims 1 to 5, characterised in that at least one of the parts of chamber (1) in contact with the gas mixture containing at least one sterilising agent has been brought to the temperature of the gas mixture containing at least one sterilising agent.

7. A process according to any one of Claims 1 to 5, characterised in that while it is carried out, the gas mixture enclosed in the chamber (1) is subjected once or several times to a pressure increase, by introducing a carrier gas or a carrier gas mixture into the chamber (1).

8. A process according to Claim 7, characterised in that the carrier gas or carrier gas mixture is nitrogen or air or a mixture of these.

9. A process according to either of Claims 7 and 8, characterised in that:
- the material or article to be sterilised is introduced into the chamber (1);
- this chamber (1) is brought to a first subatmospheric pressure;
- steam is introduced at a temperature which is equal or close to that to which the material or article is to be brought, until the intended teperature is attained, a partial suction of this steam and of the condensed water out of the chamber (1) being continuously carried out;
- the admission of steam is interrupted;
- the chamber (1) is brought to a second subatmospheric pressure until the disappearance of the water which condensed during the introduction of steam;
- the chamber (1) is brought into communication with the solution (22) which is kept at a constant temperature equal to the temperature chosen for the gas mixture which is produced by vaporising this solution (22);
- the gas mixture present in the chamber is circulated by being brought continuously into contact with the solution (22);
- after the concentration of the sterilising agent in the gas mixture present in the chamber (1) has attained equilibrium with the solution (22), a carrier gas or a carrier gas mixture is introduced into the chamber (1), thus increasing the pressure therein until a total pressure is produced which is less than, equal or greater than atmospheric pressure;
- the surface of the material or article is thus exposed for the time required to produce sterility, whilst the gas mixture present in the chamber (1) is kept in circulation and brought continuously into contact with the solution (22);
- the comunication between the solution (22) and the gas mixture present in the chamber (1) is cut;
- the sterilising agent present in the chamber (1) is removed;
- the interior of the chamber (1) is brought back to atmospheric pressure, and
- the material or article is withdrawn.

10. A process according to either of Claims 7 and 8, characterised in that:
- the material or article to be sterilised is introduced into the chamber (1);
- this chamber (1) is brought to a first subatmospheric pressure;
- steam is introduced at a temperature which is equal or close to that to which it is desired to bring the material or article, until the intended temperature is attained, a partial suction of this steam and of the condensed water out of the chamber (1) being continually produced;
- the introduction of steam remaining possible, a carrier gas or carrier gas mixture is introduced one or more times into the chamber (1), until a total pressure which is less than, equal to or

15

greater than atmospheric pressure is produced and after each introduction of carrier gas or carrier gas mixture, the chamber (1) is brought to a subatmospheric pressure which is less than that which was produced by introducing the carrier gas or carrier gas mixture;

- the admission of steam is interrupted;
- the chamber (1) is brought to a second subatmospheric pressure until the water which has condensed during the introduction of steam has disappeared;
- the chamber (1) is placed in communication with the solution (22) which is kept at a constant temperature equal to the temperature chosen for the gas mixture produced by vaporising this solution (22);
- the gas mixture present in the chamber is circulated by being brought continuously into contact with the solution (22);
- after the concentration of the sterilising agent in the gas mixture present in the chamber (1) has attained equilibrium with the solution (22), a carrier gas or carrier gas mixture is introduced into the chamber (1), thus increasing the pressure therein until total pressure which is less than, equal to or greater than atmospheric pressure is obtained;
- the surface of the material or article is thus exposed for the time required to produce sterility, whilst the gas mixture present in the chamber (1) is kept in circulation and continuously brought into contact with the solution;
- the communication between the solution (22) and the gas mixture present in the chamber (1) is cut;
- the sterilising agent present in the chamber (1) is removed;
- the interior of the chamber (1) is brought back to atmospheric pressure, and
- the material or article is withdrawn from it.

11. An apparatus for sterilising materials or articles by placing them in contact with a gas mixture containing at least one sterilising agent produced by vaporising a liquid solution (22), this apparatus comprising a fluid-tight treatment chamber (1) with constant temperature control (3, 4, 5), equipped with at least one door designed for loading materials or articles into the chamber (1) and withdrawing them therefrom, characterised in that it comprises in addition:

- a sterilising agent generator (21) suitable for containing a solution (22) of this sterilising agent and vaporising this solution (22) at a predetermined temperature by virtue of means of heating and/or cooling and means for temperature control (24, 25, 26, 27)
- suitable means (33) for introducing the gas mixture produced by the sterilising agent generator (21) into the chamber (1), for circulating the gas mixture present in the chamber (1) and for bringing it into contact with the solution (22) present in the sterilising agent generator (21);
- a vacuum source (6, 7, 8, 9) connected to the chamber (1);
- a circuit for admitting a carrier gas (10, 11, 13, 14) connected to the chamber (1) and comprising a means for sterilising a gas (10) and a means for heating this gas (14) at a constant temperature;
- a steam generator (16) which is connected, on the one hand, to the chamber (1) and, on the other hand, to a water supply (19) and which is provided with a system for controlling the temperature and/or pressure (20);
- suitable means (8, 12, 18, 29, 32) for placing the sterilising agent generator (21), the vacuum source (6, 7, 8, 9), the admission circuit for carrier gas (10, 11, 13, 14) and the steam generator (16) in communication with the chamber (1) or cutting these communications.

12. An apparatus according to Claim 11, characterised in that the chamber (1) is enclosed in a suitable enclosure (3) for circulating a heat-carrying fluid (4) and connected to suitable means (5) for ensuring the circulation, heating and temperature control of the heat carrying fluid (4).

13. An apparatus according to Claim 11, characterised in that the chamber (1) is a chamber of a plastic material which does not conduct electricity, which withstands the forces due to the subatmospheric pressures and which is equipped with electrical heating elements which are internal and/or embedded in the walls.

14. An apparatus according to any one of Claims 11 to 13, characterised in that the means for heating the gas (14) at a constant temperature in the circuit for admitting carrier gas (10, 11, 13, 14) is a heat exchanger heated by the steam generator (16).

15. An apparatus according to any one of Claims 11 to 14, characterised in that the chamber (1) is equipped with a temperature probe (35) connected to a regulating device which causes the steam generator to continue to operate at least until the load has attained a predetermined temperature.

16. An apparatus for sterilising materials or articles by placing them in contact with a gas mixture containing at least one sterilising agent produced by vaporizing a liquid solution (22), characterised in that it consists of a tunnel with constant temperature control which is provided with a suitable transporter for passing the materials or articles in succession therein and which comprises, from its entry to its exit:

- a pre-heating section equipped with suitable means for bringing the materials or articles to a predetermined temperature;
- an entry protection section equipped with suitable means for producing a flow of air preheated to a predetermined temperature, directed so as to prevent the gaseous mixture present in the following section of the tunnel (sterilisation section) from being capable of escaping through the tunnel entry;
- a sterilisation section which is equipped with a suitable sterilising agent generator (21) for containing a solution (22) of this sterilising agent and vaporising this solution (22) at a predetermined temperature, by virtue of means for heating and/or cooling and means for temperature control (24, 25, 26, 27), and which is also equipped with suitable means (33) for introducing the gas mixture produced by the sterilising agent generator (21) into the sterilisation section of the tunnel, circulating the gas mixture present in this section and bringing this gas mixture into contact with the solution (22) present in the sterilising agent generator (21);

- an exit protection section provided with suitable means for producing a flow of sterile air directed so as to prevent the gaseous mixture present in the preceding section of the tunnel (sterilisation section) from being able to escape through the exit of the tunnel.

17. An apparatus according to any one of Claims 11 to 16, characterised in that the sterilising agent generator (21) is outside the chamber (1) or tunnel.

18. An apparatus according to any one of Claims 11 to 16, characterised in that the sterilising agent generator (21) is inside the chamber or tunnel.

19. An apparatus according to any one of Claims 11 to 18, characterised in that the steam generator (16) maintains a steam pressure which is at most equal to atmospheric pressure.

20. An apparatus according to any one of Claims 11 to 19, characterised in that the means for heating and temperature control (24, 25, 26, 27) of the sterilising agent generator (21) maintain the same temperature as in the steam generator (16).

21. An apparatus according to any one of Claims 11 to 20, characterised in that the suitable means (33, 37) for introducing the gas mixture (23) produced by the sterilising agent generator (21) into the sterilisation space, for circulating the gas mixture present in this space and for bringing it into contact with the solution (22) present in the sterilising agent generator (21), comprise a liquid ring pump (37), from which the liquid is withdrawn and brought continuously into the solution (22) present in the sterilising agent generator (21).

22. An apparatus according to any one of Claims 11 to 20, characterised in that the suitable means (33, 37) for introducing the gas mixture (23) produced by the sterilising agent generator (21) into the sterilisation space, for circulating the gas mixture present in this space and for bringing it back into contact with the solution (22) present in the sterilising agent generator (21), comprise a liquid jet pump from which the liquid, which is propelled by a liquid circulation pump, is withdrawn and brought continuously into the solution (22) present in the sterilising agent generator (21).

23. An apparatus according to any one of Claims 11 to 22, characterised in that it additionally incorporates means for programming the operations of the process.

*Fig.1.*

EP 0 117 860 B2

Fig.2.

Fig.3.

*Fig. 4.*

*Fig. 5.*

9

*Fig.6.*

Temps (min)